# FASCICULE DE BREVET EUROPEEN

(11) **EP 2 934 547 B1**
(45) Date de publication et mention de la délivrance du brevet: **17.07.2019**
(21) Numéro de dépôt: 13818339.7
(22) Date de dépôt: 18.12.2013
(51) Int. Cl.: A61K 31/60, A61P 9/14, A61P 9/10

(54) **UTILISATION D'ACIDE ACÉTYLSALICYLIQUE POUR LA PRÉVENTION ET/OU LE TRAITEMENT TOPIQUE DES PLAIES DU DIABÉTIQUE**
VERWENDUNG EINER ACETYLSALICYLSÄURE ZUR TOPISCHEN PRÄVENTION UND/ODER BEHANDLUNG VON DIABETISCHEN WUNDEN
USE OF ACETYLSALICYLIC ACID TO PREVENT AND/OR TREAT TOPICALLY DIABETIC WOUNDS

(30) Priorité: 21.12.2012 FR 1262644
(43) Date de publication de la demande: 28.10.2015
(73) Titulaire: Urgo Recherche Innovation et Développement, 21300 Chenove (FR); Institut National de la Santé et de la Recherche Médicale (INSERM), 75013 Paris (FR); Institut De Recherche Pour Le Développement (IRD), 13002 Marseille 2 (FR); Université Paul Sabatier Toulouse III, 31400 Toulouse (FR)
(72) Inventeur: COSTE, Agnès, 31700 Blagnac (FR); DARDENNE, Christophe, 31470 Fontenilles (FR); PIPY, Bernard, 31400 Toulouse (FR)
(74) Mandataire: Cabinet Plasseraud
(86) Numéro de dépôt international: PCT/FR2013/053155
(87) Numéro de publication internationale: WO 2014/096697

(56) Documents cités:
- EP-A1- 0 784 975
- EP-A1- 1 547 602
- WO-A1-2010/141711
- US-A1- 2004 116 356
- PIMPLE B P ET AL: "Protective effect of Luffa acutangula extracts on gastric ulceration in NIDDM rats: Role of gastric mucosal glycoproteins and antioxidants", ASIAN PACIFIC JOURNAL OF TROPICAL MEDICINE, vol. 5, no. 8, août 2012 (2012-08), pages 610-615, XP008164223, ISSN: 1995-7645
- KONTUREK PETER C ET AL: "NO releasing aspirin (ASA) fails to impair ulcer healing in diabetic rats due to the reversal of the fall in ghrelin, leptin and COX-2 expression", GASTROENTEROLOGY, vol. 126, no. 4, Suppl. 2, avril 2004 (2004-04), page A614, XP008164225, & DIGESTIVE DISEASE WEEK/105TH ANNUAL MEETING OF THE AMERICAN-GASTROENTEROLOGICAL-ASSOCIATION; NEW ORLEANS, LA, USA; MAY 16 -20, 2004 ISSN: 0016-5085

## Description

L'objet de la présente invention consiste en une nouvelle utilisation topique d'acide acétylsalicylique, à une concentration comprise entre 200 et 4800 µM, dans la prévention et/ou le traitement des plaies du diabétique.

La cicatrisation d'une plaie est un processus physiopathologique naturel, les tissus humains et animaux étant capables de réparer des lésions localisées par des processus de réparation et de régénération qui leur sont propres.

La cicatrisation naturelle d'une plaie se déroule principalement selon trois séquences chronologiques majeures. Chacune de ces séquences est caractérisée par des activités cellulaires spécifiques et est contrôlée par une multitude de signaux de régulation (aussi bien positifs que négatifs) qui, collectivement, orchestrent et encadrent la progression du processus de réparation. On distingue ainsi:
- la phase inflammatoire ;
- la phase de prolifération (qui comprend la phase de granulation et d'épithélialisation); et
- la phase de remodelage.

La première phase, la phase inflammatoire, débute dès la rupture des vaisseaux sanguins, évènement qui déclenche la formation d'un caillot (coagulation du sang) principalement composé de fibrine et de fibronectine, et qui va constituer une matrice provisoire. Cette matrice comble en partie la lésion et va permettre la migration au sein de la zone lésée des cellules inflammatoires recrutées pour assurer la détersion de la plaie. Les plaquettes présentes vont également libérer des facteurs (par exemple des cytokines et/ou des facteurs de croissance) permettant le recrutement de cellules impliquées dans le processus cicatriciel. Cette phase se caractérise par l'infiltration et l'activation, sur le site de la lésion, de nombreuses cellules inflammatoires (polynucléaires, macrophages) assurant la défense de l'organisme contre d'éventuels micro-organismes étrangers ainsi que le nettoyage de la plaie ou détersion.

La seconde phase correspond au développement du tissu de granulation. On observe d'abord une colonisation de la blessure par migration et prolifération des fibroblastes. Puis, la migration des cellules endothéliales à partir des vaisseaux sains va permettre la néovascularisation, ou angiogenèse, du tissu lésé. Dans le tissu de granulation, les fibroblastes sont activés et vont se différencier en myofibroblastes présentant des propriétés contractiles importantes, propriétés générées par les microfilaments d'actine, permettant ainsi une contraction de la plaie. Ces microfilaments sont exprimés par une protéine : l' α-actine du muscle lisse. Ces myofibroblastes jouent un rôle majeur dans la formation et la contraction du tissu de granulation qui va conduire à la cicatrisation de la lésion. Il y a ensuite migration des kératinocytes à partir des berges de la plaie puis différenciation de ces derniers, aboutissant à la reconstruction de l'épiderme.

Cette phase de développement du tissu de granulation est initiée suite à une diminution préalable de l'état inflammatoire général de la lésion, à la disparition progressive des polynucléaires neutrophiles et à l'apparition de macrophages dont des macrophages dits réparateurs. Cette transition entre la phase inflammatoire et la phase de prolifération/réparation est appelée phase de résolution de l'inflammation.

La troisième phase du processus est une étape principalement de remodelage visant à reconstruire un tissu fonctionnel, afin que le tissu néoformé retrouve les caractéristiques et les propriétés initiales du tissu d'origine. Une partie de la matrice extracellulaire est digérée par des protéases (essentiellement des métalloprotéases matricielles et des élastases), et on observe une réorganisation de la matrice extracellulaire. Progressivement, le collagène de type III, majoritaire dans le tissu de granulation, est remplacé par le collagène de type I, principal composant matriciel du derme. A la fin de la phase de maturation, les fibroblastes, myofibroblastes et cellules vasculaires voient leur prolifération et/ou leur activité réduites. Puis, les cellules excédentaires meurent par apoptose parallèlement au remodelage de la matrice extracellulaire.

Néanmoins, certains types de plaies ne cicatrisent pas correctement, les 3 étapes clés du processus se déroulant de manière anormale et ce, malgré la mise en place des meilleures conditions physico-chimiques et biologiques possibles. En effet la rapidité et la qualité de la cicatrisation d'une plaie dépendent de facteurs intrinsèques et extrinsèques. Ce processus de réparation peut donc être anormalement prolongé selon :
- l'étiologie de la plaie ;
- son état et sa localisation ;
- la survenue d'une infection causée par la présence de certains agent infectieux comme Staphylococcus *aureus* ou Pseudomonas *aeruginosa* ;
- l'existence d'une pathologie préexistante (comme le diabète, une déficience immunitaire, une insuffisance veineuse, etc...) ;
- l'environnement extérieur ; ou
- des facteurs génétiques prédisposant ou non à des troubles de la cicatrisation.

Parmi ces plaies, on retrouve les plaies chroniques telles que les ulcères veineux, les escarres ou les plaies caractéristiques des sujets diabétiques, et plus particulièrement les plaies du pied diabétique.

Les plaies chroniques se définissent par une absence de cicatrisation après un délai de 6 semaines à compter de l'apparition de la plaie et ce quelque soit le traitement appliqué. Les plaies du diabétique sont caractérisées comme étant un type particulier et à part de plaies chroniques.

En effet, la cause première de l'absence de cicatrisation de ces plaies diabétiques est liée à une biodisponibilité du glucose exacerbée. Celle-ci induit de nombreuses modifications physiologiques et métaboliques, telles qu'un épaississement de la peau, un stress oxydatif important conduisant à une neuropathie et à une artériopathie. L'artériopathie et la neuropathie sont deux facteurs de risque majeurs de chronicisation et donc de retard de la cicatrisation des plaies diabétiques, et plus particulièrement des plaies du pied diabétique. Les autres types de plaies chroniques les plus connus, tels que les escarres ou les ulcères veineux, artériels ou mixtes, ne découlent pas de la même pathologie. Par exemple, c'est l'insuffisance veineuse qui est à l'origine de la formation, de la chronicisation et donc du retard de cicatrisation des ulcères veineux. Les escarres, quant à eux, sont des plaies consécutives à des cycles d'ischémie/reperfusion suite à une pression et une friction excessives et prolongées, exercées sur les tissus cutanés.

C'est ainsi que tous les patients diabétiques atteints de plaies sont exposés à des complications qui sont bien souvent responsables de la morbidité et de la mortalité dudit patient. Plusieurs problèmes majeurs viennent perturber le bon déroulement de la cicatrisation chez ce type de sujets. Un premier retard de cicatrisation s'opère dès la phase inflammatoire : le passage de la phase inflammatoire à la phase de prolifération est perturbé. On parle d'un problème au niveau de la résolution de l'inflammation. La phase inflammatoire est une phase essentielle de la cicatrisation mais doit être transitoire. La résolution de l'inflammation est un point critique qui conditionne l'amorçage des autres phases de la cicatrisation. Ce phénomène dynamique implique la disparition des cellules inflammatoires (polynucléaires neutrophiles) et l'apparition des macrophages. Il y a alors production de médiateurs anti-inflammatoires chimiotactiques et angiogéniques permettant notamment la migration et la différenciation des fibroblastes, cellules clés de la phase de granulation.

Une perturbation de cette phase inflammatoire, comme cela est le cas chez les sujets précités, va engendrer un prolongement anormal de la phase inflammatoire et aboutir à la chronicité de la lésion en retardant ainsi toutes les étapes ultérieures de la cicatrisation.

A terme, la phase de fermeture de la plaie, notamment lors de l'épithélialisation, s'en trouve retardée, voire, et ce dans la majorité des cas, non aboutie.

L'aspirine ou acide acétylsalicylique est connu depuis longtemps pour être considérée comme un remède efficace à bien des symptômes ou pathologies. Son mode d'action le plus répandu est une action ancestrale visant à soulager la douleur, ou encore à faire baisser la fièvre. Elle est également utilisée à faible dose pour son action préventive contre la survenue de troubles cardio-vasculaires (action anti-thrombotique).

En effet, les propriétés antiagrégants permettent de lutter efficacement contre la formation de caillot de sang dans les vaisseaux sanguins. Cependant, il semblerait que cette action soit davantage bénéfique dans le cadre d'un acte préventif suivant un premier désordre vasculaire, plus qu'en prévention primaire, où la réelle efficacité de cette substance apparaît intangible.

Il s'agit ici dans chacun des cas évoqués *supra*, d'une administration d'aspirine par voie orale. Des applications d'aspirine par voie topique existent également. En effet, l'aspirine est aussi connue par application topique pour avoir une action bénéfique sur les cicatrices hypertrophiques et notamment sur l'amélioration de l'esthétique de ces cicatrices (cf. Wound Repair Regen., 2012 Nov 5., Epub ahead of print, Topical application of a film-forming emulgel dressing that controls the release of stratifin and acetylsalicylic acid and improves/prevents hypertrophic scarring par Rahmani-Neishaboor E, Jallili R, Hartwell R, Leung V, Carr N, Ghahary A.,Department of Surgery, Division of Plastic Surgery, University of British Columbia, Vancouver, BC, Canada).

De plus, l'action positive de l'acide acétylsalicylique ou aspirine, ou d'un de ses dérivés, sur la cicatrisation d'une plaie normale, ainsi que sur la douleur de la plaie, est également connue, nonobstant l'activité antiagrégant de cette dernière, qui pourrait paraître antinomique à la destinée d'une action pro-cicatrisante.

En effet, la demande de brevet EP 1 581 252 de la société Kimberly Clark décrit une composition comprenant de l'aspirine pour stimuler la prolifération cellulaire, et plus particulièrement celle des fibroblastes et des kératinocytes. Cette composition améliore la cicatrisation d'une plaie grâce à la stimulation de la prolifération cellulaire. Ce document révèle que l'effet de l'aspirine est dose-dépendant pour des concentrations comprises entre 10⁻⁵ et 10⁻⁶ M, les doses les plus efficaces se trouvant dans les concentrations les plus faibles. La composition finale décrite comprend une concentration d'aspirine entre 1 µM et 5 mM. La stimulation de la prolifération cellulaire, à savoir celle des fibroblastes et celle des kératinocytes, ainsi que la stimulation de la production de collagène, sont des processus importants de la cicatrisation, intervenant dans les étapes de prolifération ainsi que dans la phase très tardive de remodelage.

Le demande de brevet EP 0 784 975 de la société Japonaise Teikoku décrit quant à elle une préparation pharmaceutique à base d'aspirine pour une application topique destinée à traiter des blessures de peau, mimées notamment par des modèles de rats présentant des escarres. Il est à noter que ces modèles sont des modèles d'animaux sains. En effet, aucun des rats de l'étude ne présente de troubles métaboliques de type diabète. Dans cette demande, la préparation a pour but d'augmenter la vitesse de formation du tissu de granulation mais aussi celle de la restauration de l'épiderme chez des animaux sains et présentant une ou plusieurs plaies. Ainsi cette demande s'attache à relancer le processus cellulaire de la cicatrisation dans ses phases les plus tardives.

Aucun de ces documents ne s'intéresse à la première étape de la cicatrisation, à savoir la phase inflammatoire et sa résolution, la phase inflammatoire étant pourtant une étape primordiale et indispensable puisque qu'elle initie le processus global de cicatrisation. En effet, les plaies du diabétique nécessitent une prise en charge ou une intervention plus précoce dans la cinétique du processus cicatriciel.

C'est pourquoi la Demanderesse a constaté qu'il existait un réel besoin dans la réalisation d'un traitement précoce, efficace, et ciblé des plaies du diabétique.

De façon tout à fait surprenante, la Demanderesse a pu constater que l'utilisation de l'aspirine permettait de rétablir une phase inflammatoire contrôlée, que l'on pourrait également qualifier de normale (non prolongée et non exacerbée) mais aussi d'accélérer la résolution de l'inflammation, permettant par conséquent d'aboutir à une fermeture de la plaie plus précoce et plus rapide, chez les sujets diabétiques.

La présente invention a donc pour objet l'utilisation, topique, d'acide acétylsalicylique ou d'un de ses sels, à une concentration comprise entre 200 et 4800 µM, pour son utilisation dans la prévention et/ou le traitement des plaies du diabétique.

Ainsi la présente invention a pour objet l'acide acétylsalicylique ou l'un de ses sels, à une concentration comprise entre 200 et 4800 µM, pour son utilisation pour la prévention et/ou le traitement des plaies du diabétique, l'administration de l'acide acétylsalicylique ou l'un de ses sels à cette concentration étant réalisée sous forme topique.

La présente invention a également pour objet une composition pharmaceutique comprenant de l'acide acétylsalicylique ou l'un de ses sels, dont la concentration est comprise entre 200 et 4800 µM, pour son utilisation pour la prévention et/ou le traitement des plaies du diabétique, l'administration de ladite composition étant réalisée sous forme topique.

Par « plaie du diabétique », on entend une plaie survenant chez un sujet diabétique de type I ou diabétique de type II, notamment choisie parmi les plaies des membres inférieurs, et de préférence les plaies du pied diabétique.

Par « sel d'acide acétylsalicylique », on entend un sel d'acide acétylsalicylique avec un cation pharmaceutiquement acceptable, de préférence l'acétylsalicylate de lysine ou l'acétylsalicylate de sodium.

Par « dose efficace », on entend, au sens de la présente invention, une quantité d'acide acétylsalicylique ou d'un de ses sels comprise entre 7,2.10⁻³ et 0.173 mg par plaie, préférentiellement comprise entre 7,2.10⁻³ et 0.1044 mg par plaie et préférentiellement encore entre 1,08.10⁻² et 7,2.10⁻² mg par plaie. Cette quantité d'acide acétylsalicylique ou d'un de ses sels est celle mise au contact direct de la plaie. Il s'agit d'une plaie que l'on pourrait définir comme avoisinant 1 cm² de surface. Néanmoins, la dose efficace d'acide acétylsalicylique ou d'un de ses sels varie en fonction de nombreux paramètres tels que, par exemple, la taille de la plaie, le poids, l'âge, le sexe, la sensibilité et le type d'individu (Humain ou animal) à traiter. En conséquence, la dose efficace optimale devra être déterminée en fonction des paramètres jugés pertinents, par le spécialiste en la matière.

Par « chronicisation », on entend tout retard dans la cicatrisation desdites plaies.

Par « traiter » les plaies du diabétique, on entend une cicatrisation et une fermeture desdites plaies.

Par « prévenir » les plaies du diabétique, on entend empêcher le retard de cicatrisation ou chronicisation desdites plaies.

L'acide acétylsalicylique ou l'un de ses sels est utilisé dans l'invention à une concentration efficace. En effet, utilisé à une concentration inférieure à 200 µM, par exemple à 100 µM, l'acide acétylsalicylique ou l'un de ses sels n'a pas d'effet sur l'accélération de la vitesse de cicatrisation des plaies du diabétique.

De même, à une concentration supérieure ou égale à 5000 µM l'acide acétylsalicylique n'a plus d'action bénéfique sur l'accélération de la cicatrisation des plaies diabétiques, voire même une action néfaste sur la viabilité de certaines cellules présentes au niveau de la plaie, comme par exemple les macrophages.

Selon un mode de mise en oeuvre préféré, l'acide acétylsalicylique ou l'un de ses sels est utilisé à une concentration comprise entre 200 et 2900 µM.

Selon un mode de mise en oeuvre encore plus préféré, l'acide acétylsalicylique ou l'un de ses sels est utilisé à une concentration comprise entre 300 et 2000 µM.

De préférence, la concentration définie d'acide acétylsalicylique ou l'un de ses sels est la concentration dispensée au contact de la plaie.

De préférence, l'acide acétylsalicylique ou l'un de ses sels est utilisé pour le traitement de la chronicisation des plaies du diabétique. En particulier, l'acide acétylsalicylique ou l'un de ses sels permet de rétablir une phase inflammatoire dite contrôlée (non prolongée et non exacerbée), d'accélérer la résolution de l'inflammation, et par voie de conséquence d'accélérer la cinétique de fermeture de la plaie.

De préférence, l'acide acétylsalicylique ou un de ses sels selon l'invention est formulé dans un milieu physiologiquement acceptable, afin d'obtenir une composition pharmaceutique. Par « milieu physiologiquement acceptable », on entend un milieu compatible avec la peau, les plaies, les muqueuses et les phanères.

De préférence, l'acide acétylsalicylique ou l'un de ses sels est associé à un lipide ou à une huile hyperoxygénée, ou à un de leurs dérivés. Ledit lipide ou ladite huile hyperoxygénée peut être formulé dans la composition pharmaceutique comprenant déjà l'acide acétylsalicylique ou un de ses sels, ou bien peut être administré indépendamment de l'acide acétylsalicylique ou un de ses sels. De préférence, le lipide est un acide gras de type Omega-3 ou Omega-6 ou une association des deux.

Par « Omega-3 » on entend tout composé choisi parmi la liste non limitative constituée de l'acide α-linolénique, l'acide eicosapentaénoïque ou encore de l'acide docosahexaénoïque.

Par « Omega-6 » on entend tout composé choisi parmi la liste non limitative constituée de l'acide linoléique, l'acide γ-linolénique, l'acide eicosadiénoïque, l'acide dihomo-γ-linolénique, l'acide arachidonique, l'acide docosadiénoïque, l'acide docosatétraénoïque ou encore de l'acide docosapentaénoïque.

Par huile « hyperoxygénée », on entend toute huile d'origine végétale hyperoxygénée, la définition de cette huile en spectrophotométrie UV étant de 10 à 20 pour le facteur E270 et de 8 à 60 pour le facteur E232.

De préférence, l'huile d'origine végétale hyperoxygénée présente un taux de peroxydation compris entre 30 et 300 exprimé en milliéquivalents par kilogramme et une teneur en glycérides oxydés comprise entre 5 et 40.

De préférence encore, l'huile d'origine végétale hyperoxygénée présente un taux de peroxydation compris entre 50 et 150 exprimé en milliéquivalents par kilogramme.

Selon un mode de réalisation préféré, l'huile d'origine végétale hyperoxygénée est choisie parmi le groupe constitué par les huiles de maïs, les huiles d'amande douce, les huiles de carthame, les huiles de noisette, les huiles d'arachide, les huiles d'olive, les huiles de colza, les huiles de soja, les huiles d'Onagre, les huiles de tournesol, les huiles de pépin de raisin, les huiles de sésame et de leurs mélanges, et préférentiellement une huile de maïs.

L'acide acétylsalicylique ou un de ses sels, et la composition pharmaceutique les comprenant, sont administrés par voie locale, appelée également voie topique. L'administration par voie locale ou topique implique que le principe actif, en l'occurrence l'acide acétylsalicylique ou un de ses sels, agisse de manière locale après absorption par la peau, la plaie ou les muqueuses. Diverses formes galéniques existent, telles que des solutions, des émulsions, des crèmes, des pommades, des lotions, des patchs, des gels, des pansements, des microcapsules, des microfibres, des sprays, des poudres ou des nanofibres.

De préférence, l'acide acétylsalicylique ou l'un de ses sels est compris dans une composition (pharmaceutique) choisie parmi les pansements, les microcapsules, les microfibres, les nanofibres, les solutions, les lotions, les gels, les patchs, les émulsions, les crèmes, les pommades, les poudres et les sprays.

Lorsque la composition pharmaceutique selon l'invention est sous forme d'une émulsion, elle comprend au moins une phase aqueuse et/ou une phase huileuse, et un agent tensio-actif. En effet, les émulsions classiques sont des systèmes instables quasi-homogènes de deux liquides non miscibles, dont l'un est dispersé dans l'autre sous forme de fines gouttelettes (micelles). Cette dispersion est stabilisée grâce à l'action d'agents tensio-actifs qui modifient la structure et le rapport des forces au niveau de l'interface, et donc augmentent la stabilité de la dispersion en diminuant l'énergie de tension interfaciale.

La composition pharmaceutique selon l'invention peut également se présenter sous forme de gel ; elle comprend alors un ou plusieurs composés gélifiants.

Lorsque la composition pharmaceutique se présente sous forme de solution, elle comprend, outre l'acide acétylsalicylique ou l'un de ses sels, une solution aqueuse ou huileuse, et éventuellement un ou plusieurs solvants et/ou propénétrants de l'acide acétylsalicylique ou l'un de ses sels.

La composition pharmaceutique selon l'invention pourra en outre comprendre des additifs inertes ou des combinaisons de ces additifs, tels que :
- des agents épaississants ou émulsifiants ;
- des agents régulateurs de pH;
- des filtres UV-A et UV-B;
- et des antioxydants.

Les agents épaississants ou émulsifiants peuvent être choisis parmi la liste non limitative de composés comprenant notamment la gomme de xanthane ou encore la famille des tensioactifs.

Les agents régulateurs de pH peuvent être choisis parmi la liste non limitative de composés comprenant notamment les carbonates.

Les filtres UV-A et UV-B peuvent être choisis parmi la liste non limitative de composés comprenant les filtres chimiques et minéraux. Parmi les filtres chimiques, on trouve l'octocrylène, les benzophénones, le drométrizole trisiloxane ou encore l'avobenzone. Parmi les filtres minéraux, on trouve l'oxyde de zinc ou le dioxyde de titane.

Les antioxydants peuvent être choisis parmi la liste non limitative de composés comprenant notamment l'acétate de tocophérol ou l'acide ascorbique.

Bien entendu, l'homme du métier veillera à choisir le ou les éventuels composés à ajouter à ces compositions pharmaceutiques de telle manière que les propriétés avantageuses attachées intrinsèquement à la présente invention ne soient pas ou substantiellement pas altérées par l'addition envisagée.

Ces additifs peuvent être présents dans la composition de 0,001 à 20% en poids par rapport au poids total de la composition.

Il va être maintenant donné, à titre d'illustration les exemples suivants.

D'autres caractéristiques et avantages de l'invention apparaîtront plus clairement à la lecture de la description qui suit d'un mode de réalisation préféré de l'invention, donnée à titre d'exemple dont voici les légendes des figures :
Figure 1 : Cinétique de fermeture de la plaie des souris « contrôle » et diabétiques (HFD et db/db)
Figure 2 : Evaluation de l'exsudat et quantification du nombre de cellule dans l'exsudat des souris « contrôle » et diabétiques (HFD et db/db). Les légendes du tableau sont les suivantes :
   Absence d'exsudat :-
   Suintement : +/-
   Exsudation faible : +
   Exsudation modérée : ++
   Exsudation forte : +++
Figure 3 : Quantification du nombre total de polynucléaires neutrophiles et de macrophages dans l'exsudat des souris « contrôle » et diabétiques (HFD)
Figure 4 : Cinétique de fermeture de la plaie des souris « témoin » et diabétiques (db/db) traitées par l'acide acétylsalicylique
Figure 5 : Cinétique de fermeture de la plaie des souris « témoin » et diabétiques (db/db) traitées par l'acide acétylsalicylique
Figure 6 : Viabilité des macrophages *in vitro* en présence d'acide acétylsalicylique
Figure 7 : Cinétique d'infiltration cellulaire dans la plaie des souris « témoin » et diabétiques (HFD) traitées par l'acide acétylsalicylique.

### EXEMPLES

### MATERIEL & METHODES

### Souris db/db :

Les souris homozygotes db/db présentent une mutation ponctuelle dans le gène du récepteur à la leptine, hormone qui contrôle notamment le phénomène de satiété. Les souris db/db, deviennent hyperphagiques et spontanément obèses à partir de leurs 3^{ème}/4^{ème} semaines de vie. Ces animaux sont caractérisés par une hyperglycémie, une résistance à l'insuline et une hypertriglycéridémie, faisant alors de ces souris des modèles diabétiques d'origine génétique de premier choix. Les souris du groupe « contrôle » des souris db/db sont les souris hétérozygotes db/+.

### Souris HFD :

Les souris HFD sont des modèles murins de diabète induit réalisés avec des souris C57BL/6 âgées de 8 semaines. Ces souris suivent un régime alimentaire hypercalorique (hyperlipidique) durant 16 semaines. Elles présentent une forte prise de poids due à l'augmentation de leur tissu adipeux et développent également une intolérance au glucose ainsi qu'une résistance à l'insuline. Contrairement aux souris db/db, l'état diabétique de ces animaux n'est pas d'origine génétique. Les souris du groupe « contrôle » des souris HFD sont des souris sous régime standard, également connues sous l'abréviation « NC ».

Tous les animaux sont maintenus selon un cycle journalier divisé en 12 heures d'obscurité suivi de 12h de luminosité à 22°C avec un accès perpétuel à l'eau et à la nourriture. La mise en place du diabète chez les souris est validée selon deux tests permettant d'évaluer le métabolisme du glucose : le test de tolérance au glucose (IPGTT) et le test de sensibilité à l'insuline (IPIST).

### Suivi de la cinétique de cicatrisation :

La souris est anesthésiée à l'aide d'isofluorane (gaz anesthésique halogéné). La partie dorsale et les flancs de la souris sont tondus, désinfectés et une excision circulaire proche de 1 cm² est effectuée. Le tissu connectif situé à l'interface du tissu musculaire est supprimé sans endommager le fascia musculaire. Cette excision totale de la peau ainsi réalisée va jusqu'au panniculus carnosus.

La plaie ainsi réalisée est protégée par un dispositif tel que décrit dans les demandes de brevets déposées par les Laboratoires Urgo FR 11 62295 et FR 11 62344, permettant à la fois de protéger la plaie, ainsi que d'observer et d'évaluer la cinétique de la cicatrisation et la récolte des exsudats.

Le processus de cicatrisation des plaies est observé de 0 à 30 jours post lésion. Pour chaque animal, entre 3-5 photographies numériques standardisées à très grande résolution sont prises. Le pourcentage de fermeture des plaies est basé sur les changements d'aires calculés pour la même blessure, sur la même souris, et pour les points de temps indiqués. Ainsi, la moyenne calculée pour la cicatrisation des plaies (en pourcentage) parmi les 3 souris dans un groupe est obtenue indépendamment, et les statistiques sont calculées à partir de 3 groupes de 3 expériences indépendantes.

Les cellules de l'exsudat sont récupérées de J1 post lésion jusqu'à ce que plus d'exsudats ne soient sécrétés par la plaie. Afin de déterminer la cinétique d'infiltration des polynucléaires neutrophiles, les cellules de l'exsudat de la lésion sont caractérisées par un immuno-marquage portant sur les récepteurs membranaires Ly6G, 7/4 (pour les polynucléaires) et F4/80 (pour les macrophages) en cytométrie en flux. Un total de 10 000 événements est analysé pour chaque échantillon. Tous les résultats sont rapportés sous forme de pourcentage d'expression.

### RESULTATS OBTENUS

Les résultats sont exprimés en moyenne ± la SEM. La SEM désigne l'écart standard de la moyenne. Elle représente l'écart de la moyenne de l'échantillon par rapport à la moyenne de la vraie population. Elle est calculée en divisant l'écart type par la racine carrée de la taille de l'échantillon. Les analyses statistiques sont réalisées selon la méthode de comparaison multiple, test de Bonferroni-Dunnet portant au minimum sur 3 sujets pour chaque groupe (n=3). P<0,05 (*) est considéré comme statistiquement significatif.

### A. Suivi de la cicatrisation

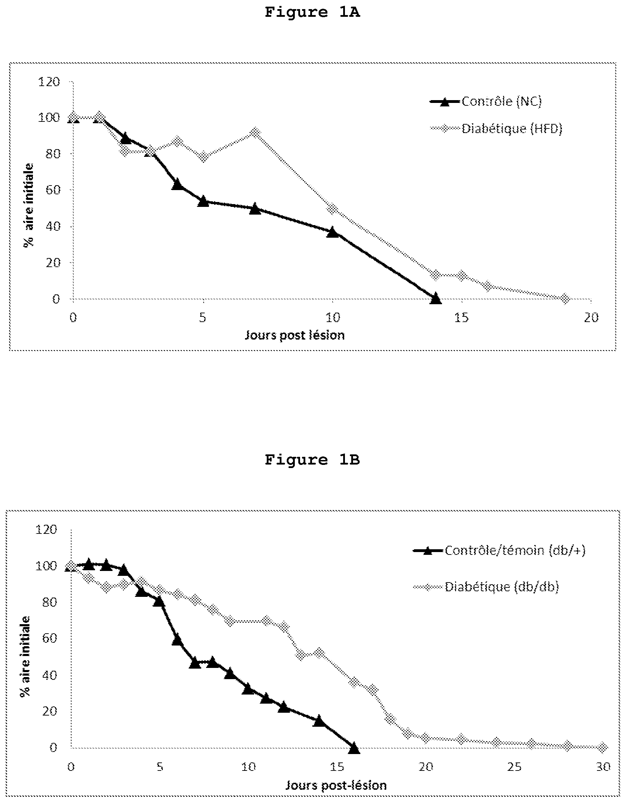

### Cinétique de fermeture de la plaie (figures 1A et 1B) :

Chez les souris « contrôle », on observe la fermeture totale de la lésion en 14 jours. En revanche chez les souris HFD, cette fermeture n'est totale qu'après une durée de 18 jours, soit 4 jours de retard effectif (figure 1A).

Chez les souris db/db, la fermeture totale de la plaie n'est effective qu'entre J26 à J28, soit 10 à 12 jours de retard de cicatrisation par rapport à leur contrôle respectif (figure 1B).

Il y a donc un retard significatif de la fermeture de la plaie chez les souris diabétiques (HFD et db/db).

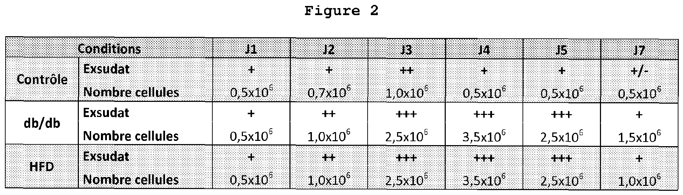

### Observation macroscopique de la cicatrisation (exsudats + tissu de granulation) (figure 2):

Chez les souris « contrôle », suite à la création de la lésion cutanée, on observe la mise en place d'un tissu de granulation qui apparaît progressivement au niveau de la plaie. Son expansion n'est pas homogène mais prend son origine en différents points des berges de la plaie pour la combler entièrement en 5 jours. Ce tissu s'épaissit au fur et à mesure de la progression de la cicatrisation.

Les souris diabétiques présentent un net retard dès le deuxième jour post-lésion dans la mise en place de ce tissu de granulation. Le comblement total de la lésion n'intervient seulement qu'après 7 jours post-lésion chez les souris diabétiques, soit deux jours supplémentaires en comparaison aux souris « contrôle ».

Chez les souris diabétiques, la mise en place du tissu de granulation s'accompagne d'un phénomène d'exsudation. Ce liquide, riche en cellules et médiateurs, est produit dès la création de la lésion. Chez les souris « contrôle », le pic de production est atteint dès J3 post lésion.

En revanche, les souris diabétiques (HFD et db/db) présentent une exsudation beaucoup plus importante en termes de volumes dès J2 post lésion mais surtout qui perdure dans le temps (figure 2).

Il y a un donc un retard significatif dans la mise en place du tissu de granulation chez les souris diabétiques accompagné d'une exsudation plus importante et persistante.

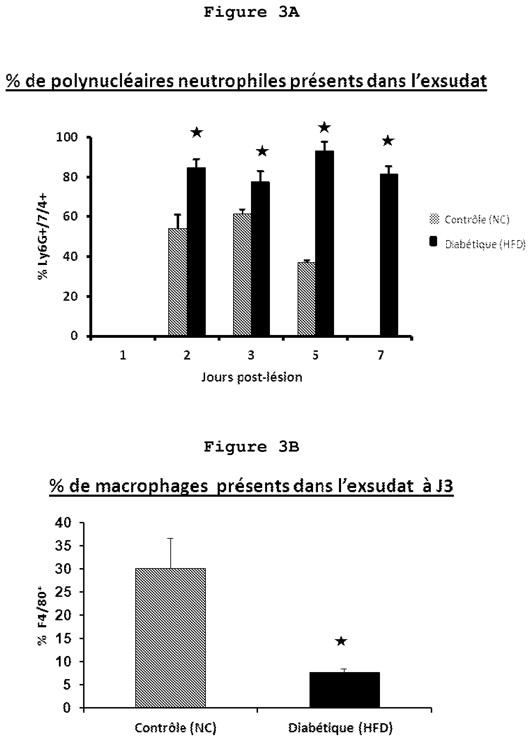

### B. Analyse des populations cellulaires présentes dans l'exsudat (Figures 2, 3A et 3B)

Les souris « contrôle » présentent de manière générale un volume d'exsudat faible et l'infiltration cellulaire dans cet exsudat est limitée. Une infiltration cellulaire dans l'exsudat est constatée le deuxième jour post lésion et ce jusqu'à 5 jours post-lésion. Un pic d'infiltration cellulaire est constaté à J3 post-lésion (figure 2)
La cinétique d'infiltration cellulaire dans l'exsudat des souris diabétiques est différente de celle observée chez les souris « contrôle ». En effet on observe une infiltration cellulaire, plus importante qui persiste dans le temps jusqu'à J7 post-lésion (figure 2).

La différence d'infiltration cellulaire que l'on constate en fonction du temps s'explique par la persistance des polynucléaires neutrophiles sur le site de la lésion des souris diabétiques. En effet, chez les souris « contrôle », les polynucléaires neutrophiles disparaissent au bout de 5 jours post-lésion alors que chez les souris diabétiques, cette population cellulaire persiste dans le temps, jusqu'à J7 post-lésion (figure 3A). Ainsi, il apparaît clairement que le pourcentage de polynucléaires infiltrant la lésion cutanée est beaucoup plus important pour le groupe diabétique que pour le groupe « contrôle ».

Les souris diabétiques présentent une infiltration cellulaire plus importante prenant son origine dans la persistante de polynucléaires neutrophiles sur le site de la lésion.

Les macrophages arrivent sur le site de la lésion dès J3 post-lésion chez les souris « contrôle ». A l'opposé, cette infiltration de cellules n'est que faiblement visible à J3 post-lésion chez les souris diabétiques, sans augmenter durant la poursuite de la mise en place du tissu de granulation (figure 3B).

Les souris diabétiques présentent une infiltration cellulaire plus importante qui s'explique :
- Par la persistante de polynucléaires neutrophiles sur le site de la lésion ;
- Par un retard dans le recrutement des macrophages ; et
- Par un défaut de disparition de ces polynucléaires.

### RESULTATS OBTENUS CHEZ LES SOURIS TRAITEES AVEC L'ACIDE ACETYLSALICYLIQUE

### A.Suivi de la cinétique de cicatrisation de souris diabétiques en présence d'acide acétylsalicylique utilisé à différentes concentrations

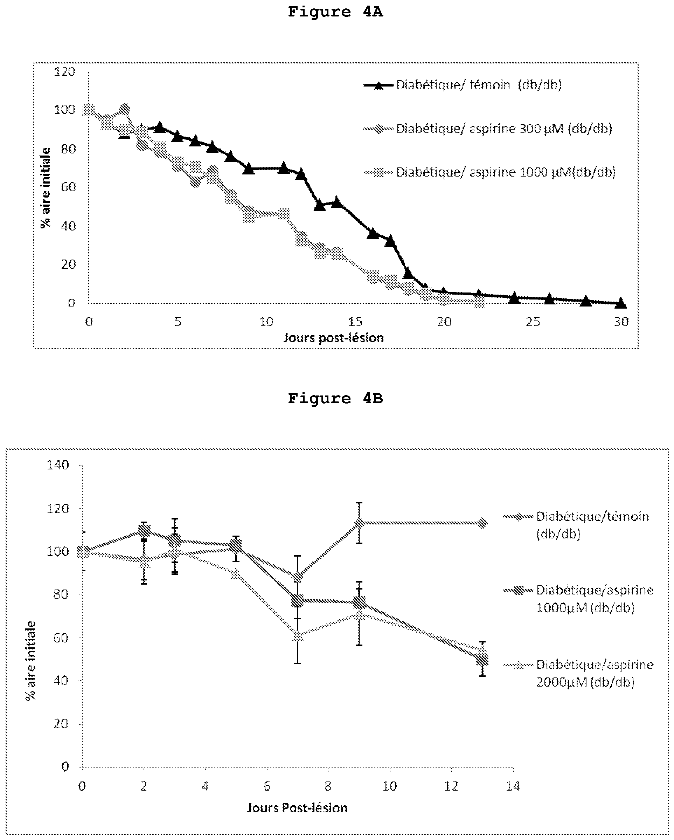

### Cinétique de fermeture de la plaie (figures 4, 5 et 6) :

Comme déjà décrit précédemment, la souris est anesthésiée à l'aide d'isofluorane. La partie dorsale et les flancs de la souris sont tondus, désinfectés et une excision circulaire proche de 1 cm² est effectuée. Le tissu connectif situé à l'interface du tissu musculaire est supprimé sans endommager le fascia musculaire. Cette excision totale de la peau ainsi réalisée va jusqu'au panniculus carnosus.

Entre temps, la solution de référence d'acide acétylsalicylique est préparée à 5 mM. Cette solution sera utilisée pour préparer les solutions aux concentrations 100 µM, 200µM, 300 µM, 1 mM, 2mM, 2,9 mM, 4,8 mM et 5 mM.

Des solutions à des concentrations de 100 µM, 200µM, 300 µM, 1 mM, 2mM, 2,9 mM, 4,8 mM, et 5mM d'acide acétylsalicylique sont testées.

Par souci de précision, un tableau de correspondance entre les concentrations d'acide acétylsalicylique et les doses administrées au niveau de la plaie est établi, voir *infra.* Ces doses administrées au niveau de la plaie correspondent à un mode de réalisation particulier de l'invention.

**Table 1 :**

| Concentration en acide acétylsalicylique testée sur la plaie (pour une plaie avoisinant 1 cm2) | Dose d'acide acétylsalicylique administrée sur la plaie (pour une plaie avoisinant 1 cm2) |
|---|---|
| 100 µM | 0.0036 mg |
| 200 µM | 0.0072 mg |
| 300 µM | 0.0108 mg |
| 1 mM | 0.036 mg |
| 2 mM | 0.072 mg |
| 2,9 mM | 0.1044 mg |
| 4,8 mM | 0.173 mg |
| 5 mM | 0.2 mg |

A partir de chaque solution, un volume compris entre 50 et 500 µL est prélevé, et mis au contact de la plaie par l'intermédiaire d'un dispositif tel que décrit au travers des demandes de brevets déposées par les Laboratoires Urgo FR 11 62295 et FR 11 62344.

Il est à noter que cette mise en contact d'une concentration prédéfinie d'acide acétylsalicylique sur le site cicatriciel est réalisée à compter du troisième jour post-lésion. En effet, c'est à cette période que le retard de cicatrisation apparait chez les souris diabétiques, comparativement aux souris « contrôle » (figure 1A et 1B). Par ailleurs, dans le cadre du traitement de patients diabétiques, les plaies traitées seront déjà formées et stagnantes en phase inflammatoire. Aucune plaie chronique, et plus particulièrement les plaies du diabétique, ne sont traitées dès leur apparition. De plus, la phase inflammatoire est une phase indispensable à la cicatrisation, puisqu'elle permet le nettoyage de la zone lésée et sa détersion grâce à l'infiltration de cellules inflammatoires comme les polynucléaires neutrophiles. Il est donc nécessaire de conserver une inflammation afin d'initier une cicatrisation efficace, et donc de ne pas bloquer complètement cette phase. C'est pourquoi le traitement par l'acide acétylsalicylique n'est proposé qu'à partir de J3 post-lésion.

Les figures 4A et 4B décrivent les cinétiques de cicatrisation des plaies de souris diabétiques après application alternativement d'une solution de NaCl (correspondant à notre « témoin »), ou d'une solution à 300 µM ou à 1000 µM d'acide acétylsalicylique, ou d'une solution à 2000 µM.

Les souris diabétiques traitées à 300 µM, 1000 µM et 2000 µM d'acide acétylsalicylique présentent des cinétiques de cicatrisation totalement différentes de celles des souris « témoin ». Alors que chez les souris diabétiques « témoin » la fermeture de la plaie se conclue en 26 jours, les souris diabétiques traitées avec 300 µM et 1000 µM d'acide acétylsalicylique voient leurs plaies se refermer aux alentours de vingt jours post-lésion. Par analogie, la figure 4B, laisse entrevoir les mêmes résultats pour une dose de 2000 µM.

De plus, nous pouvons observer que l'accroissement de la vitesse de fermeture des plaies traitées par les solutions d'acide acétylsalicylique à 300 µM, 1000 µM et 2000 µM se fait par l'intermédiaire d'une accélération dans la phase précoce de la cicatrisation (phase inflammatoire) à savoir entre J3 et J7 post-lésion, visible sur la figure 4. Cette accélération de la cicatrisation entre J3 et J7 post-lésion est à corréler avec une vitesse de résolution de l'inflammation accélérée. Ainsi la vitesse de cicatrisation, et donc la vitesse de fermeture de la plaie des souris sur lesquelles sont administrées lesdites solutions d'acide acétylsalicylique sont fortement accélérées par rapport aux souris « témoin ».

En résumé l'administration topique de solutions à 300 µM, 1000 µM et 2000 µM d'acide acétylsalicylique rétablit une phase inflammatoire contrôlée et induit une très nette accélération de la résolution de l'inflammation permettant ainsi une augmentation de la vitesse de fermeture de la plaie (environ à J20 post-lésion) comparativement aux souris diabétiques « témoin ».

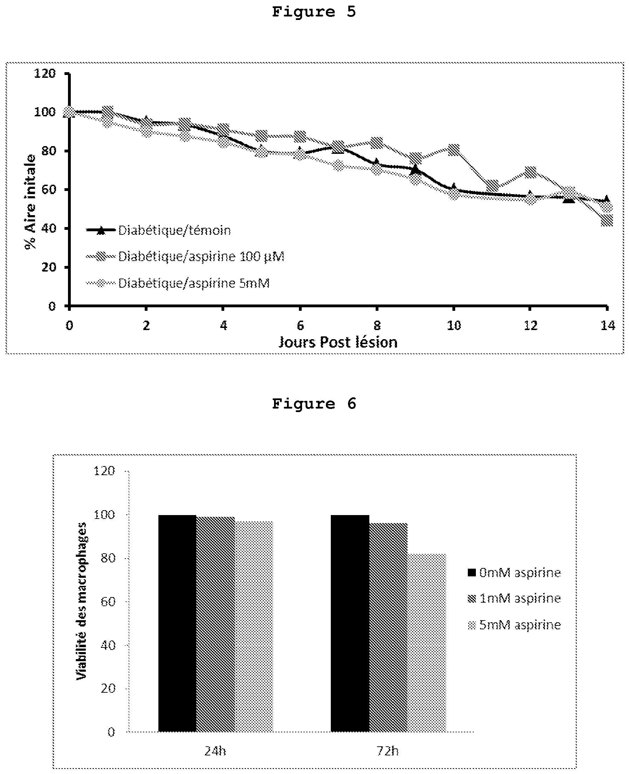

La figure 5 décrit les cinétiques de cicatrisation des plaies de souris diabétiques après application alternativement d'une solution de NaCl (correspondant à notre « témoin »), ou d'une solution à 100 µM ou à 5000 µM d'acide acétylsalicylique.

Les cinétiques de cicatrisation des souris diabétiques « témoin » et des souris diabétiques traitées à 100 µM et à 5000 µM d'acide acétylsalicylique sont similaires et ne présentent pas de différences significatives. On ne constate pas le moindre effet suite à l'introduction de telles concentrations d'acide acétylsalicylique au contact de la plaie sur la cinétique de cicatrisation par rapport aux souris diabétiques « témoin », et surtout pendant la phase inflammatoire ainsi que pendant la mise en place du tissu de granulation.

En résumé, l'administration topique de solutions d'acide acétylsalicylique à 100 µM et 5000 µM sur les plaies de souris diabétiques ne fait preuve d'aucune efficacité sur le rétablissement d'une phase inflammatoire contrôlée ni sur l'accélération de la résolution de l'inflammation et ne permettant pas ainsi une augmentation de la vitesse de fermeture de la plaie.

De plus des tests de viabilité cellulaire sont réalisés pour différentes concentrations en acide acétylsalicylique sur des macrophages (figure 6).

Cette méthode d'analyse comprend l'utilisation de monocytes dérivés de sang périphérique humain qui sont mis en culture dans du milieu RPMI dans lequel est additionné du M-CSF à 10 ng/ml pendant 24 heures afin de les différencier en macrophages. L'acide acétylsalicylique est ajouté dans le milieu de culture à raison de 1000 µM ou 5000 µM final.

La mortalité cellulaire est analysée après 72 heures d'incubation par cytométrie en flux, par mesure de l'incorporation cytoplasmique d'iodure de propidium.

Le but d'un tel test de viabilité cellulaire réalisé *in vitro* est de vérifier la nocivité de la dose administrée sur les cellules retrouvées au niveau de la plaie, et de façon préférentielle dans le présent cas sur les macrophages, cellules considérées comme un type de cellules effectrices dans la cicatrisation de la lésion étudiée. En effet, une telle étude est impossible sur les polynucléaires neutrophiles du fait de leur mortalité basale trop importante *in vitro.*

La figure 6 révèle une très nette perte de viabilité cellulaire pour une dose de 5000 µM d'acide acétylsalicylique administrée sur les cellules *in vitro* à 72h, avec une valeur de viabilité qui se trouve diminué de 20 %, et donc que l'on peut caractériser comme nocive.

A l'inverse, l'application d'une dose de 1000 µM ne révèle aucune toxicité cellulaire.

Ainsi la solution à une concentration de 5 mM administrée sur la plaie est d'une part non efficace sur la cinétique de cicatrisation (figure 5) et en plus se révèle nocive pour les cellules présentes sur le site cicatriciel (figure 6).

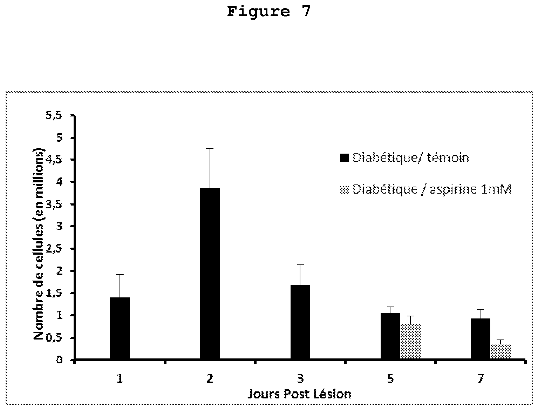

### B.Analyse des populations cellulaires présentes dans l'exsudat (figure 7)

La figure 7 décrit la cinétique d'infiltration cellulaire réalisée sur des souris diabétiques « témoin » ou sur lesquelles se trouvent appliquée une solution de 1000 µM d'acide acétylsalicylique.

A J7 post-lésion, on remarque que le traitement à l'acide acétylsalicylique permet de diminuer significativement le nombre de cellules infiltrées sur le site de la lésion chez les souris diabétiques traitées.

On peut donc conclure à la lecture de l'ensemble des résultats précédents que le traitement à base d'aspirine à une concentration comprise entre 100 µM et 4800 µM (administrées dés J3 post-lésion) permet de diminuer l'infiltration cellulaire chez les souris diabétiques, de restaurer une phase inflammatoire contrôlée, et d'accélérer la résolution de l'inflammation pour permettre à termes d'augmenter la vitesse de fermeture de la plaie.

L'acide acétylsalicylique a donc un rôle crucial dans le processus de résolution de l'inflammation et par voie de conséquence sur la vitesse de fermeture de la plaie de l'individu diabétique.

## Revendications

1. Acide acétylsalicylique ou l'un de ses sels, à une concentration comprise entre 200 et 4800 µM, pour son utilisation pour la prévention et/ou le traitement des plaies du diabétique, l'administration de l'acide acétylsalicylique ou l'un de ses sels à cette concentration étant réalisée sous forme topique.

2. Acide acétylsalicylique ou l'un de ses sels pour son utilisation selon la revendication 1, **caractérisé en ce que** la concentration d'acide acétylsalicylique ou l'un de ses sels est comprise entre 200 et 2900 µM.

3. Acide acétylsalicylique ou l'un de ses sels pour son utilisation selon la revendication 1 ou 2, **caractérisé en ce que** la concentration d'acide acétylsalicylique ou l'un de ses sels est comprise entre 300 et 2000 µM.

4. Acide acétylsalicylique ou l'un de ses sels pour son utilisation selon l'une quelconque des revendications précédentes, **caractérisé en ce que** le sel d'acide acétylsalicylique est choisi parmi l'acétylsalicylate de lysine et l'acétylsalicylate de sodium.

5. Acide acétylsalicylique ou l'un de ses sels pour son utilisation selon l'une quelconque des revendications précédentes **caractérisé en ce que** l'acide acétylsalicylique ou l'un de ses sels est compris dans une composition choisie parmi les pansements, les microcapsules, les microfibres, les nanofibres, les solutions et les sprays.

6. Acide acétylsalicylique ou l'un de ses sels pour son utilisation selon l'une quelconque des revendications précédentes, pour le traitement de la chronicisation des plaies du diabétique.

7. Acide acétylsalicylique ou l'un de ses sels pour son utilisation selon l'une quelconque des revendications précédentes, pour accélérer la résolution de l'inflammation.

8. Acide acétylsalicylique ou l'un de ses sels pour son utilisation selon l'une quelconque des revendications précédentes, **caractérisé en ce que** l'acide acétylsalicylique ou l'un de ses sels est associé à un lipide, une huile hyperoxygénée ou à un de leurs dérivés.

9. Acide acétylsalicylique ou l'un de ses sels pour son utilisation selon la revendication 8, **caractérisé en ce que** le lipide ou un de ses dérivés est un acide gras de type Omega-3 ou Omega-6, ou une association des deux.

10. Composition pharmaceutique comprenant de l'acide acétylsalicylique ou l'un de ses sels, dont la concentration est comprise entre 200 et 4800 µM, pour son utilisation pour la prévention et/ou le traitement des plaies du diabétique, l'administration de ladite composition étant réalisée sous forme topique.

## Patentansprüche

1. Acetylsalicylsäure oder eines ihrer Salze, mit einer Konzentration von 200-4800µM) zur Anwendung in der Prävention und/oder der Behandlung von diabetischen Wunden, wobei die Gabe der Acetylsalicylsäure oder eines ihrer Salze in dieser Konzentration in topischer Form erfolgt.

2. Acetylsalicylsäure oder eines ihrer Salze zur Anwendung nach Anspruch 1, **dadurch gekennzeichnet, dass** die Konzentration der Acetylsalicylsäure oder eines ihrer Salze 200-2900 µM) beträgt.

3. Acetylsalicylsäure oder eines ihrer Salze zur Anwendung nach Anspruch 1 oder 2, **dadurch gekennzeichnet, dass** die Konzentration der Acetylsalicylsäure oder eines ihrer Salze 300-2000 µM) beträgt.

4. Acetylsalicylsäure oder eines ihrer Salze zur Anwendung nach einem der vorhergehenden Ansprüche, **dadurch gekennzeichnet, dass** das Salz der Acetylsalicylsäure gewählt ist aus Lysinacetylsalicylat und Natriumacetylsalicylat.

5. Acetylsalicylsäure oder eines ihrer Salze zur Anwendung nach einem der vorhergehenden Ansprüche, **dadurch gekennzeichnet, dass** die Acetylsalicylsäure oder eines ihrer Salze in einer Zusammensetzung enthalten ist, die gewählt ist aus Verbänden, Mikrokapseln, Mikrofasern, Nanofasern, Lösungen und Sprays.

6. Acetylsalicylsäure oder eines ihrer Salze zur Anwendung nach einem der vorhergehenden Ansprüche, zur Behandlung der Chronifizierung von diabetischen Wunden.

7. Acetylsalicylsäure oder eines ihrer Salze zur Anwendung nach einem der vorhergehenden Ansprüche, zur Beschleunigung der Auflösung der Entzündung.

8. Acetylsalicylsäure oder eines ihrer Salze zur Anwendung nach einem der vorhergehenden Ansprüche, **dadurch gekennzeichnet, dass** die Acetylsalicylsäure oder eines ihrer Salze mit einem Lipid, einem sauerstoffangereichertem Öl oder einem von derne Derivaten kombiniert ist.

9. Acetylsalicylsäure oder eines ihrer Salze zur Anwendung nach Anspruch 8, **dadurch gekennzeichnet, dass** das Lipid oder eines seiner Derivate eine Fettsäure vom Typ Omega 3 oder Omega 6 ist, oder eine Kombination aus beiden.

10. Pharmazeutische Zusammensetzung, welche Acetylsalicylsäure oder eines ihrer Salze umfasst, mit einer Konzentration von 200-4800 µM, zur Anwendung in der Prävention und/oder der Behandlung von diabetischen Wunden, wobei die Gabe der Zusammensetzung in topischer Form erfolgt.

## Claims

1. Acetylsalicylic acid or a salt thereof, at a concentration of between 200 and 4800 µM, for use thereof in preventing and/or treating diabetic wounds the administration of the acetylsalicylic acid or a salt thereof at said concentration being carried out in topical form.

2. The acetylsalicylic acid or a salt thereof, for use thereof, as claimed in claim 1, **characterized in that** the concentration of acetylsalicylic acid or a salt thereof is between 200 and 2900 µM.

3. The acetylsalicylic acid or a salt thereof, for use thereof, as claimed in claim 1 or 2, **characterized in that** the concentration of acetylsalicylic acid or a salt thereof is between 300 and 2000 µM.

4. The acetylsalicylic acid or a salt thereof, for use thereof, as claimed in any one of the preceding claims, **characterized in that** the salt of acetylsalicylic acid is selected from lysine acetylsalicylate and sodium acetylsalicylate.

5. The acetylsalicylic acid or a salt thereof, for use thereof, as claimed in any one of the preceding claims, **characterized in that** the acetylsalicylic acid or a salt thereof is included in a composition selected from dressings, microcapsules, microfibers, nanofibers, solutions and sprays.

6. The acetylsalicylic acid or a salt thereof, for use thereof, as claimed in any one of the preceding claims, for treating the chronification of diabetic wounds.

7. The acetylsalicylic acid or a salt thereof, for use thereof, as claimed in any one of the preceding claims, for accelerating resolution of inflammation.

8. The acetylsalicylic acid or a salt thereof, for use thereof, as claimed in any one of the preceding claims, **characterized in that** the acetylsalicylic acid or a salt thereof is combined with a lipid, a hyperoxygenated oil or one of their derivatives.

9. The acetylsalicylic acid or a salt thereof, for use thereof, as claimed in claim 8, **characterized in that** the lipid or a derivative thereof is an omega-3 or omega-6 fatty acid, or a combination of the two.

10. A pharmaceutical composition comprising acetylsalicylic acid or a salt thereof, the concentration of which is between 200 and 4800 µM, for use thereof in preventing and/or treating diabetic wounds, said administration being carried out in topical form .
